# FASCICULE DE BREVET EUROPEEN

(11) **EP 2 674 122 B1**
(45) Date de publication et mention de la délivrance du brevet: **02.05.2018**
(21) Numéro de dépôt: 13171154.1
(22) Date de dépôt: 07.06.2013
(51) Int. Cl.: A61B 17/64

(54) **DISPOSITIF FIXATEUR DE BASSIN**
BEFESTIGUNGSVORRICHTUNG FÜR BECKEN
PELVIS FIXING DEVICE

(30) Priorité: 13.06.2012 FR 1255547
(43) Date de publication de la demande: 18.12.2013
(73) Titulaire: Lape Medical, 74950 Scionzier (FR)
(72) Inventeur: GRADEL, Thomas, 74970 Marignier (FR)
(74) Mandataire: Cabinet Poncet

(56) Documents cités:
- WO-A2-2006/126167
- CH-A1- 703 584
- US-A- 5 196 012
- US-A- 5 350 378

## Description

La présente invention concerne un dispositif fixateur de bassin destiné à enserrer le bassin fracturé d'un patient pour le stabiliser.

De façon connue, un dispositif fixateur de bassin comprend un premier et un deuxième bras disposés à coulissement par des moyens de glissière permettant un éloignement relatif est un rapprochement relatif desdits bras selon une première direction. A l'utilisation, le patient est en position couchée sur le dos, et les bras du fixateur sont disposés latéralement sur chaque côté du bassin.

Un tel dispositif fixateur de bassin est par exemple commercialisé par la société SYNTHES GmbH sous la dénomination « Pelvic C-Clamp ».

L'utilisation de ce dispositif fixateur de bassin par un unique opérateur s'avère toutefois complexe et difficile. En effet, la mise en place de ce dispositif fixateur de bassin sur le patient en position couchée nécessite le déplacement successif et itératif de chacun des bras pour venir enserrer le patient. Chaque déplacement de bras nécessitant l'usage des deux mains de l'opérateur, l'opérateur est contraint de procéder à des prises et lâchers successifs et itératifs de chacun des bras, tout en en se déplaçant de part et d'autre du patient. Cela induit un risque de déplacements intempestifs du dispositif fixateur de bassin par rapport au patient et nuit à son bon positionnement. Ce risque est augmenté par les déplacements de l'opérateur de part et d'autre du patient.

Ainsi, en pratique, la mise en place de ce dispositif fixateur de bassin est généralement effectuée par deux opérateurs placés de part et d'autre du patient. Cela augmente le nombre d'intervenants et nécessite un environnement largement dégagé autour du patient. En outre, les deux opérateurs doivent alors redoubler de vigilance pour synchroniser leurs gestes et éviter là encore des déplacements intempestifs du dispositif fixateur de bassin par rapport au patient.

Pour permettre un ajustement fin et précis de la position relative des bras afin d'immobiliser de façon satisfaisante un bassin fracturé, le dispositif « Pelvic C-Clamp » a recours à des manchons tubulaires destinés à respectivement s'engager à coulissement sur des broches de Kirschner, lesdits manchons tubulaires étant reliés par une liaison hélicoïdale vissée aux extrémités libres des bras. Après un positionnement relatif des bras assez approximatif, l'opérateur procède au serrage final du bassin par vissage des manchons tubulaires jusqu'à ce qu'une extrémité de chaque manchon tubulaire vienne en appui contre le bassin. Ces opérations de vissage obligent une nouvelle fois l'opérateur à se déplacer de part et d'autre du patient, à moins d'avoir recours à deux opérateurs placés de chaque côté du patient.

Enfin, le dispositif « Pelvic C-Clamp » présente un encombrement important, de sorte qu'il est fourni en plusieurs éléments démontés qu'il faut assembler avant utilisation. Ceci constitue une perte de temps et induit un risque d'erreur dans le montage du dispositif.

Le document WO 2006/126167 A1 déposé au nom de la société SYNTHES GmbH correspond au dispositif fixateur de bassin exploité sous la dénomination « Pelvic C-Clamp ». Dans un mode de réalisation particulier, ce document prévoit des moyens autorisant un déplacement dans un sens de chacun des bras par rapport au rail de guidage supérieur, tout en s'opposant à un déplacement dans le sens opposé.

Ces moyens sont maintenus en permanence activés par des moyens élastiques de rappel, et ne sont ainsi pas sélectivement activables vers des états d'embrayage et de débrayage au sens de la présente invention. Le dispositif du document WO 2006/126167 A1 présente ainsi toujours l'inconvénient d'obliger l'opérateur, lors de la mise en place de ce dispositif fixateur de bassin sur le patient, à déplacer successivement et de façon itérative chacun des bras par rapport au rail de guidage supérieur pour venir enserrer le patient, chaque déplacement de bras nécessitant l'usage des deux mains de l'opérateur (l'une pour tenir un bras, l'autre pour tenir le rail de guidage supérieur). Les risques de déplacements intempestifs du dispositif fixateur de bassin par rapport au patient et de mauvais positionnement persistent.

Un problème proposé par l'invention est de concevoir un dispositif fixateur de bassin facile à utiliser pour une mise en place simple et rapide par un unique opérateur.

Selon un autre aspect, l'invention vise à concevoir un dispositif fixateur de bassin qui peut être rangé avant et après utilisation avec un faible encombrement.

Pour atteindre ces objets ainsi que d'autres, l'invention propose un dispositif fixateur de bassin tel que défini dans la revendication 1, destiné à enserrer le bassin fracturé d'un patient pour le stabiliser. Un tel dispositif peut être rangé avec les bras rapprochés au mieux pour limiter l'encombrement global du dispositif, puis il peut être déployé par simple traction manuelle relative des bras pour les écarter et les positionner de part et d'autre du patient. L'écartement relatif des bras par traction manuelle est totalement libre avant embrayage des moyens de verrouillage unidirectionnel.

Après embrayage des moyens de verrouillage unidirectionnel, la traction manuelle relative des bras est inhibée, et la mise en place se poursuit par une simple poussée manuelle relative pour réaliser un pré-positionnement par rapprochement relatif des bras pour les faire enserrer le patient.

Lors du déploiement et du pré-positionnement du dispositif selon l'invention sur le patient, l'opérateur peut manoeuvrer le dispositif avec une main positionnée sur chacun des bras, et il n'a pas besoin de lâcher ni reprendre ceux-ci pour régler et verrouiller leur écartement relatif.

Avantageusement, les moyens d'embrayage peuvent être conformés de façon à activer les moyens de verrouillage unidirectionnel vers l'état d'embrayage lorsque que les bras sont écartés relativement selon une distance d'écartement prédéterminée, la distance d'écartement prédéterminée étant de préférence la distance d'écartement maximale des bras.

Les moyens de verrouillage unidirectionnel s'embrayent ainsi automatiquement sans que l'opérateur ne lâche l'un des deux bras du dispositif. Cet embrayage ne nécessite aucun mouvement autre qu'un simple écartement relatif des bras, mouvement qui est volontairement effectué par l'opérateur et qui est de toute façon nécessaire pour la mise en place du dispositif sur le patient. L'activation des moyens d'embrayage se fait ainsi naturellement et au bon moment. Il ne se produit aucun embrayage intempestif des moyens de verrouillage unidirectionnel à un moment inopportun ou gênant.

Selon l'invention
- les moyens de verrouillage unidirectionnel comportent un cliquet coopérant avec une tige à crémaillère,
- ledit cliquet est déplaçable entre une position d'engagement dans laquelle il est en prise dans la crémaillère de la tige et une position de libération dans laquelle il est dégagé de la crémaillère,
- des moyens de rappel élastiques rappellent en permanence le cliquet vers sa position d'engagement. Les moyens d'embrayage comportent des moyens de maintien déplaçables entre une position de maintien, dans laquelle ils maintiennent le cliquet en position de dégagement à l'encontre des moyens de rappel élastiques, et une position d'effacement, dans laquelle le cliquet peut librement se déplacer vers sa position d'engagement sous l'effet des moyens de rappel élastiques.

Les moyens de maintien empêchent l'embrayage des moyens de verrouillage unidirectionnel jusqu'à leur effacement qui laisse le cliquet s'engager librement dans la crémaillère sous l'effet des moyens de rappel élastiques.

Avantageusement, les moyens d'embrayage peuvent comporter un ergot porté par la tige à crémaillère et qui, lorsque les bras ont été écartés relativement selon une distance d'écartement prédéterminée, déplace les moyens de maintien vers leur position d'effacement.

Le seul mouvement d'écartement relatif des bras suffit ainsi à libérer le cliquet et à embrayer les moyens de verrouillage unidirectionnel.

De préférence, le dispositif peut comporter des moyens d'entraînement par vissage capables de provoquer un rapprochement relatif des bras.

Les moyens d'entraînement par vissage permettent un rapprochement relatif final lent et fin des bras pour achever leur positionnement correct de part et d'autre du patient avec une force de maintien du bassin satisfaisante.

Avantageusement, on peut prévoir que le dispositif comporte :
- une première tige à crémaillère solidaire du premier bras,
- une deuxième tige à crémaillère solidaire du deuxième bras,
lesdites première et deuxième tiges étant chacune disposée à coulissement par rapport à un manchon central.

Le dispositif comporte ainsi une structure de moyens de glissière capables d'être disposés dans une position de rangement peu encombrante tout en présentant une grande dimension de déploiement. Cela évite d'avoir à démonter le dispositif fixateur de bassin dans l'attente d'une utilisation, ou d'avoir à livrer le dispositif fixateur à l'état démonté.

En pratique, on peut prévoir que :
- le manchon central comporte un premier manchon d'extrémité et un deuxième manchon d'extrémité filetés extérieurement, reliés l'un à l'autre par un tube creux fileté intérieurement,
- l'un des manchons d'extrémité comporte des premiers moyens de verrouillage unidirectionnel de l'une des tiges à crémaillère, tandis que l'autre des manchons d'extrémité comporte des deuxièmes moyens de verrouillage unidirectionnel de l'autre des tiges à crémaillère,
- les filetages sont conformés de façon qu'une rotation du tube creux dans un premier sens provoque un rapprochement relatif des premier et deuxième manchons d'extrémité tandis qu'une rotation du tube creux dans un deuxième sens, opposé au premier sens, provoque un éloignement relatif des premier et deuxième manchons d'extrémité.

De préférence, on peut prévoir que :
- le dispositif comporte des manchons tubulaires destinés à respectivement recevoir à coulissement une broche médicale,
- les bras comportent des extrémités libres à moyens de réception conformés pour recevoir et tenir les manchons tubulaires orientés selon une deuxième direction parallèle à la première direction,
- les extrémités libres des bras sont munies de moyens d'engagement permettant une pénétration des manchons tubulaires dans les moyens de réception par un mouvement de translation selon une troisième direction perpendiculaire à la première direction.

L'engagement sur les manchons tubulaires des extrémités libres des bras par un simple mouvement de translation peut être effectué de façon simple et rapide par un unique opérateur positionné d'un seul côté du patient, sans obliger ledit opérateur à tourner autour du patient pour effectuer une visée.

Pour ce faire, en pratique, les moyens d'engagement peuvent comporter une fente à l'extrémité libre des bras.

Avantageusement, on peut prévoir que :
- les manchons tubulaires comportent un premier tronçon de diamètre extérieur réduit et un deuxième tronçon de diamètre extérieur plus important reliés par un épaulement à première face de butée,
- les moyens de réception comportent un premier tronçon de diamètre intérieur réduit et un deuxième tronçon de diamètre intérieur plus important reliés par un épaulement à deuxième face de butée,
- le premier tronçon des moyens de réception a un diamètre intérieur supérieur au diamètre du premier tronçon de manchon tubulaire et inférieur au diamètre du deuxième tronçon de manchon tubulaire,
- le deuxième tronçon des moyens de réception a un diamètre intérieur supérieur au diamètre du deuxième tronçon de manchon tubulaire.

Les manchons tubulaires sont destinés à être engagés à coulissement sur des broches médicales, dites broches de Kirschner, fixées sur le bassin du patient. La réception des premiers tronçons des manchons tubulaires dans les moyens de réception respectifs des bras procure un positionnement approximatif mais suffisant des bras par rapport aux manchons tubulaires. L'opérateur procède alors à un rapprochement relatif supplémentaire des bras jusqu'à contact de la première face de butée des manchons tubulaires avec la deuxième face de butée des moyens de réception.

De préférence, le dispositif peut comporter des moyens de verrouillage bidirectionnel aptes à s'opposer à un écartement relatif et à un rapprochement relatif des bras selon la première direction.

De tels moyens de verrouillage bidirectionnel permettent une immobilisation parfaite du dispositif fixateur de bassin en fin de mise en place sur le patient.

D'autres objets, caractéristiques et avantages de la présente invention ressortiront de la description suivante de modes de réalisation particuliers, faite en relation avec les figures jointes, parmi lesquelles :
- la figure 1 est une vue en perspective d'un dispositif fixateur de bassin selon un mode de réalisation de l'invention, ledit dispositif étant disposé dans une position de faible encombrement avant utilisation ;
- la figure 2 est une vue en perspective du dispositif de la figure 1 au cours d'un mouvement d'écartement relatif des bras ;
- la figure 3 est une vue en perspective en détail du dispositif de la figure 2 ;
- la figure 4 est une vue en coupe du dispositif de la figure 3 ;
- la figure 5 est une vue en perspective d'un détail du dispositif de la figure 1 en fin d'écartement relatif des bras ;
- la figure 6 est une vue en coupe du détail du dispositif de la figure 5 au cours d'un rapprochement relatif des bras après activation des moyens d'embrayage des moyens de verrouillage unidirectionnel ;
- la figure 7 est une vue en perspective du détail du dispositif de la figure 6 ;
- les figures 8 et 9 sont des vues en perspective illustrant la pénétration de manchons tubulaires dans des moyens d'engagement des bras ;
- la figure 10 est une vue en perspective illustrant la pénétration d'un manchon tubulaire dans les moyens d'engagement d'un bras ;
- la figure 11 est une vue en perspective du dispositif de la figure 1 en fin de positionnement ; et
- la figure 12 est une vue en coupe d'une partie du dispositif de la figure 1.

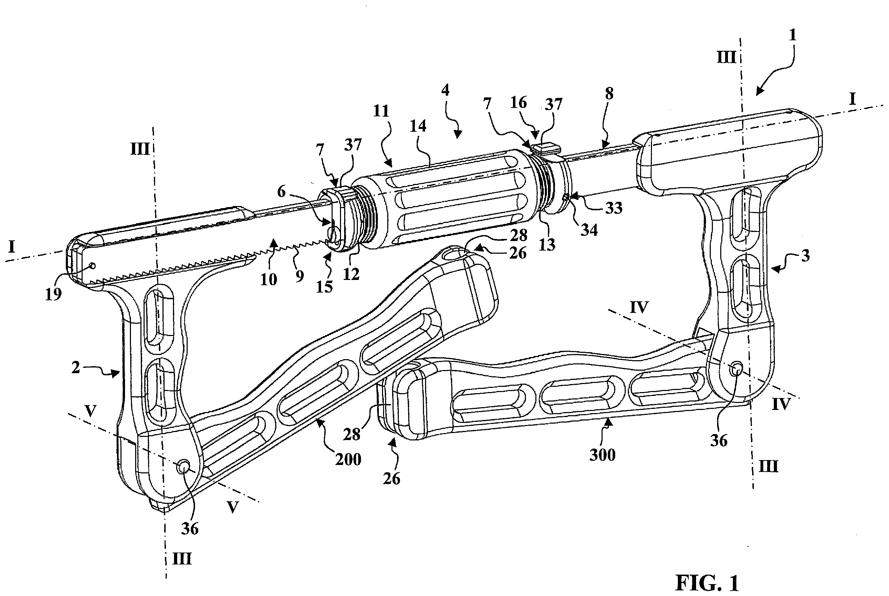

Sur la figure 1 est illustré un dispositif 1 fixateur de bassin destiné à enserrer le bassin fracturé d'un patient pour le stabiliser. Ce dispositif 1 comprend un premier bras 2 et un deuxième bras 3 disposés à coulissement par des moyens de glissière 4 permettant un éloignement relatif et un rapprochement relatif des bras 2 et 3 selon une première direction I-I.

Dans le mode de réalisation illustré sur les figures 1 à 12, le dispositif 1 comporte une première tige 8 à crémaillère 9 solidaire du premier bras 2 et une deuxième tige 10 à crémaillère 9 solidaire du deuxième bras 3. Les première et deuxième tiges 8 et 10 sont chacune disposées à coulissement par rapport à un manchon central 11.

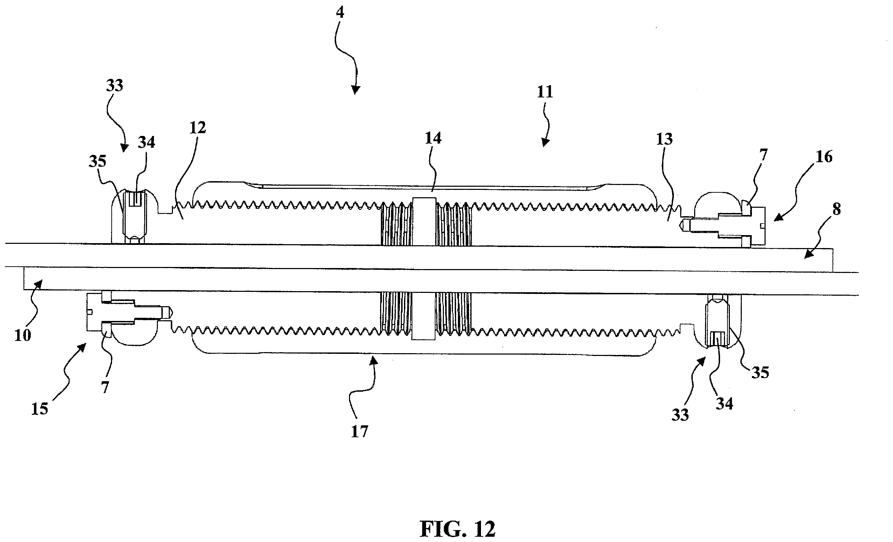

La structure du manchon central 11 est plus particulièrement illustrée sur la figure 12. Sur cette figure, on voit que le manchon central 11 comporte un premier manchon d'extrémité 12 et un deuxième manchon d'extrémité 13 filetés extérieurement, reliés l'un à l'autre par un tube 14 creux fileté intérieurement.

Les filetages des premier et deuxième manchons d'extrémité 12 et 13 et le filetage du tube 14 sont conformés de façon qu'une rotation du tube 14 creux dans un premier sens provoque un rapprochement relatif des premier et deuxième manchons d'extrémité 12 et 13, tandis qu'une rotation du tube 14 creux dans un deuxième sens provoque un éloignement relatif des premier et deuxième manchons d'extrémité 12 et 13. On dispose ainsi de moyens d'entraînement par vissage 17 capables de provoquer un rapprochement ou un écartement relatifs des premier et deuxième manchons d'extrémité 12 et 13.

Le premier manchon d'extrémité 12 comporte des premiers moyens de verrouillage unidirectionnel 15, tandis que le deuxième manchon d'extrémité 13 comporte des deuxièmes moyens de verrouillage unidirectionnel 16.

Les moyens de verrouillage unidirectionnel 15 et 16 sont activables vers un état dit d'embrayage dans lequel les moyens de verrouillage unidirectionnel 15 et 16 autorisent un rapprochement relatif des bras 2 et 3 tout en s'opposant à un écartement relatif des bras 2 et 3.

Des moyens d'embrayage 6 sont associés aux moyens de verrouillage unidirectionnel 15 et 16 et permettent de sélectivement activer vers l'état d'embrayage les moyens de verrouillage unidirectionnel 15 et 16 depuis un état dit de débrayage dans lequel les moyens de verrouillage unidirectionnel 15 et 16 autorisent un rapprochement relatif et un écartement relatif des bras 2 et 3.

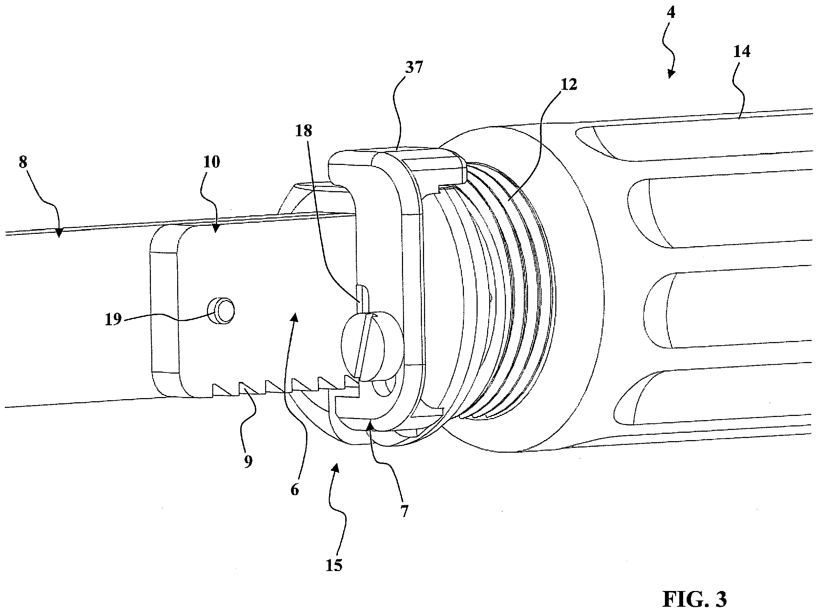

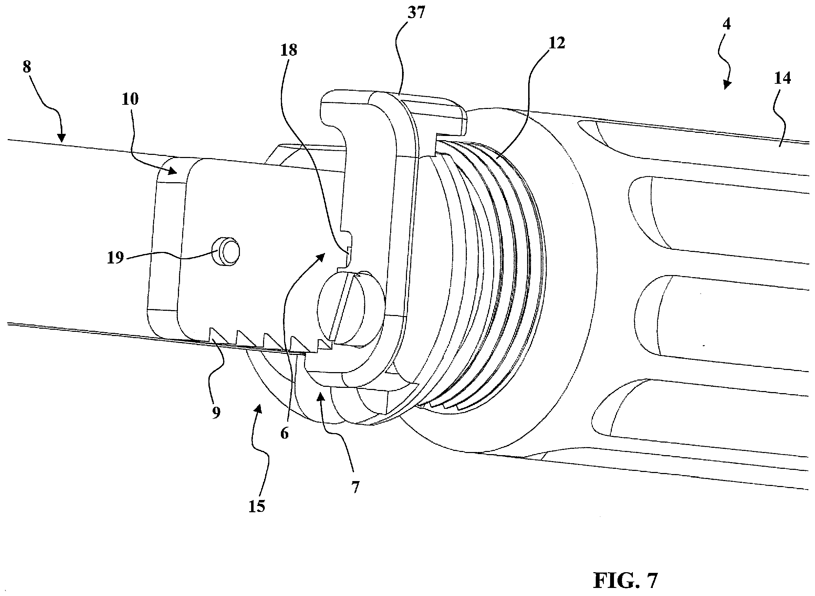

Sur les figures 3 et 7, on voit que les premiers moyens de verrouillage unidirectionnel 15 comportent un cliquet 7 coopérant avec la tige 10 à crémaillère 9. Le cliquet 7 est déplaçable entre une position d'engagement (figure 7) dans laquelle il est en prise dans la crémaillère 9 de la tige 10 et une position de libération (figure 3) dans laquelle il est dégagé de la crémaillère 9. Des moyens de rappel élastiques rappellent en permanence le cliquet 7 vers sa position d'engagement. Il en est de même pour les deuxièmes moyens de verrouillage unidirectionnel 16 qui comportent également un cliquet 7 coopérant avec la tige 8 à crémaillère 9.

Les moyens de verrouillage unidirectionnel 15 et 16 permettent un rapprochement relatif rapide des bras 2 et 3 par un unique opérateur tenant dans chacune de ses mains les bras 2 et 3 et leur appliquant un effort tendant à les rapprocher l'un de l'autre.

Les moyens d'embrayage 6 des premiers moyens de verrouillage unidirectionnel 15 sont plus particulièrement visibles sur les figures 3 à 7.

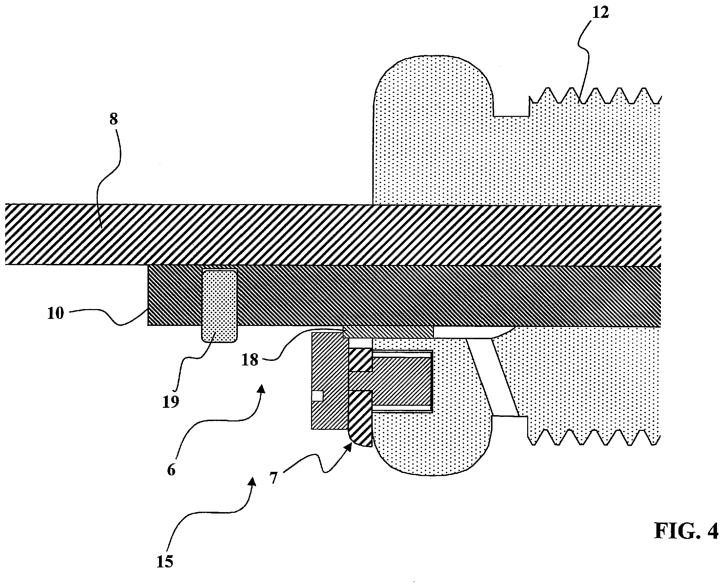

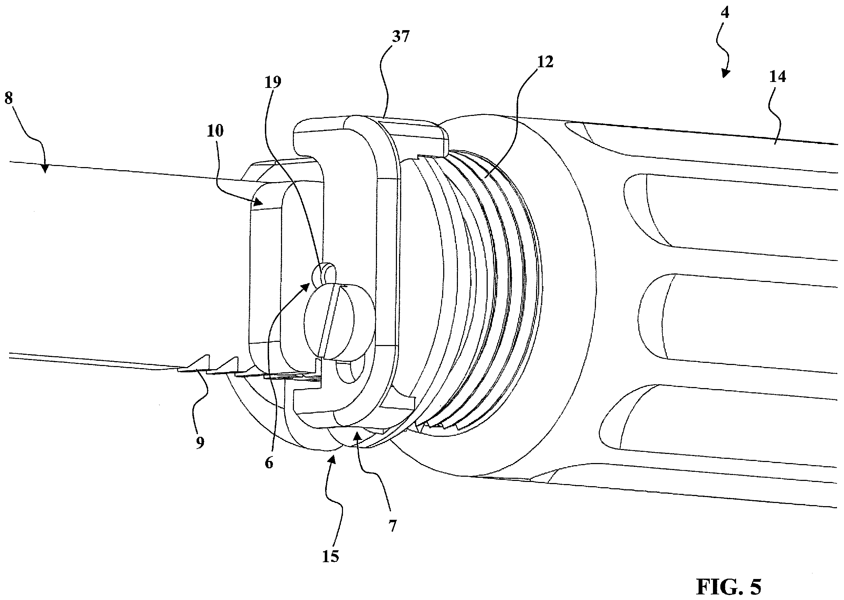

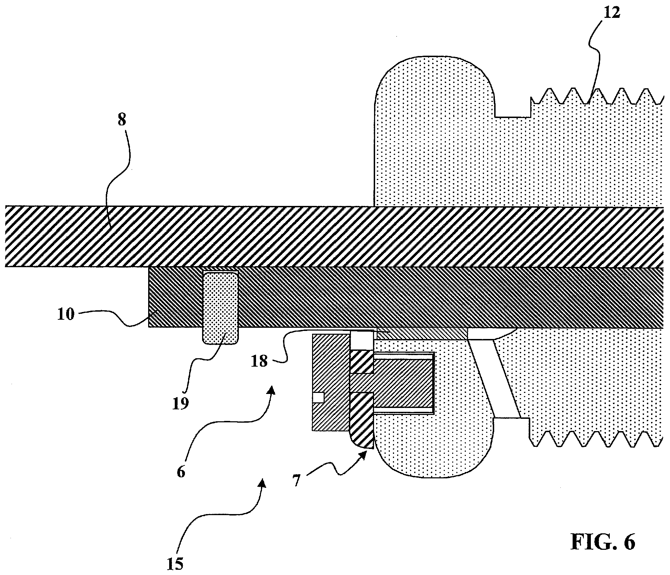

Les moyens d'embrayage 6 comportent des moyens de maintien 18 déplaçables entre une position de maintien (figures 3 et 4), dans laquelle ils maintiennent le cliquet 7 en position de dégagement à l'encontre des moyens de rappel élastiques, et une position d'effacement (figures 5 et 6), dans laquelle le cliquet 7 peut librement se déplacer vers sa position d'engagement sous l'effet des moyens de rappel élastiques.

Les moyens d'embrayage 6 comportent un ergot 19 porté par la tige 10 à crémaillère 9. Lorsque les bras 2 et 3 sont écartés relativement selon une distance d'écartement prédéterminée (figure 5), l'ergot 19 déplace les moyens de maintien 18 en position d'effacement (figures 6 et 7).

Les deuxièmes moyens de verrouillage unidirectionnel 16 comportent également des moyens d'embrayage 6, identiques à ceux des premiers moyens de verrouillage unidirectionnel 15, coopérant avec la crémaillère 9 de la tige 8.

Le dispositif 1 fixateur de bassin est destiné à coopérer avec des broches médicales, dites broches de Kirschner, fixées dans l'os du bassin du patient, de part et d'autre du bassin du patient.

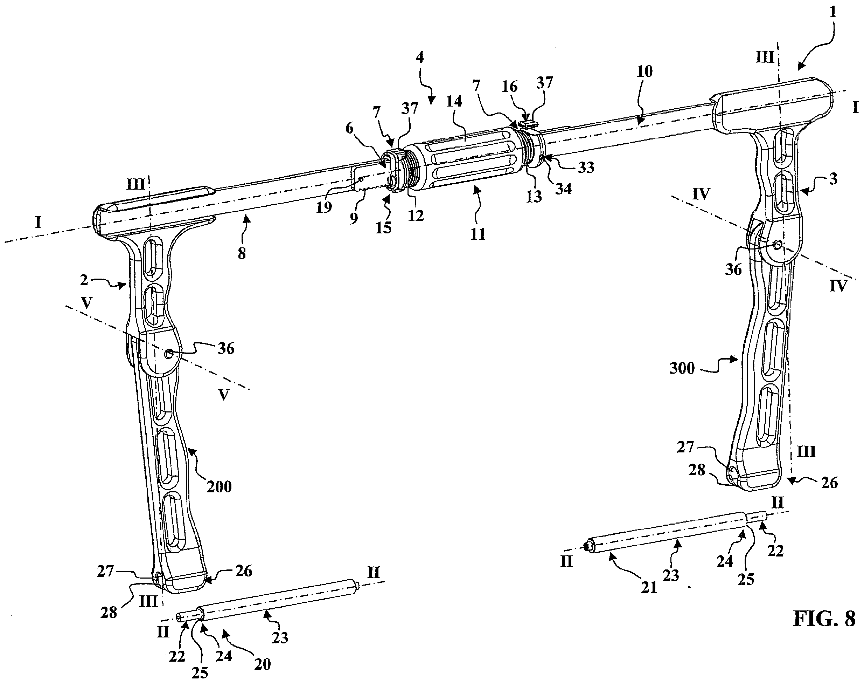

Cette coopération s'effectue à l'aide de manchons tubulaires 20 et 21, illustrés sur les figures 8 à 11, destinés à respectivement recevoir à coulissement une broche médicale.

Les manchons tubulaires 20 et 21 comportent chacun un premier tronçon 22 de diamètre extérieur réduit et un deuxième tronçon 23 de diamètre extérieur plus important reliés par un épaulement 24 à première face de butée 25.

Les manchons tubulaires 20 et 21 sont destinés à se fixer aux extrémités libres des bras 2 et 3. Pour ce faire, les bras 2 et 3 comportent chacun une extrémité libre à moyens de réception 26 conformés pour recevoir et tenir les manchons tubulaires 20 et 21 respectifs orientés selon une deuxième direction II-II parallèle à la première direction I-I. Les extrémités libres des bras 2 et 3 sont munies de moyens d'engagement 27 permettant une pénétration des manchons tubulaires 20 et 21 dans les moyens de réception 26 par un mouvement de translation selon une troisième direction III-III perpendiculaire à la première direction I-I.

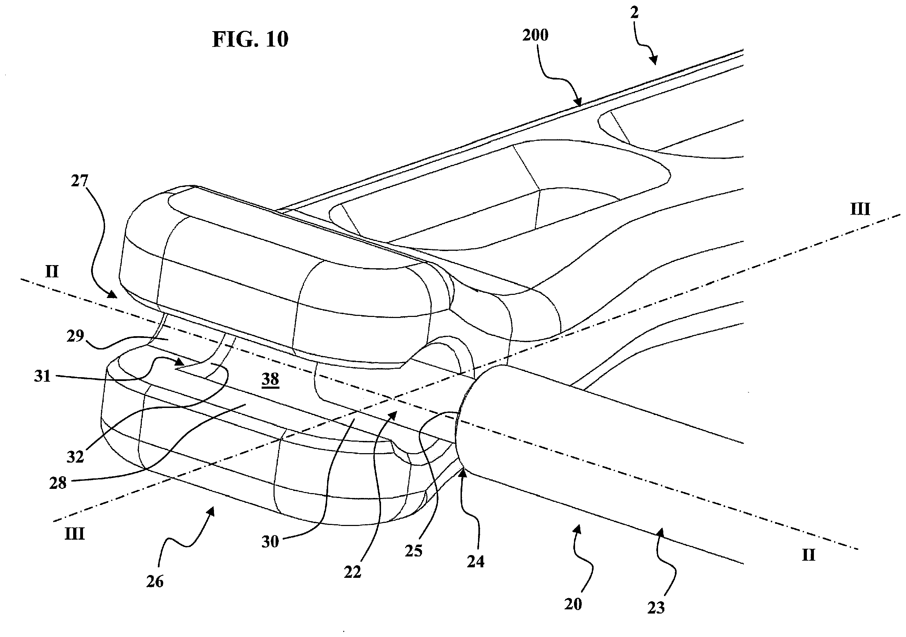

Comme il est plus visible sur la figure 10, les moyens d'engagement 27 comportent une fente 28 à l'extrémité libre du bras 2. Il en est de même pour le bras 3.

Les moyens de réception 26 de chaque extrémité libre des bras 2 et 3 comportent un premier tronçon 29 de diamètre intérieur réduit et un deuxième tronçon 30 de diamètre intérieur plus important reliés par un épaulement 31 à deuxième face de butée 32. Le premier tronçon 29 a un diamètre intérieur supérieur au diamètre du premier tronçon 22 des manchons tubulaires 20 et 21, et inférieur au diamètre du deuxième tronçon 23 des manchons tubulaires 20 et 21. Le deuxième tronçon 30 a un diamètre intérieur supérieur au diamètre du deuxième tronçon 23 des manchons tubulaires 20 et 21.

Les moyens de réception 26 sont ainsi conformés pour recevoir et tenir les manchons tubulaires 20 et 21 orientés selon la deuxième direction II-II une fois les première et deuxième face de butée 25 et 32 en appui l'une contre l'autre.

Le dispositif 1 comporte également des moyens de verrouillage bidirectionnel 33 aptes à s'opposer à un écartement relatif et à un rapprochement relatif des bras 2 et 3 selon la première direction I-I, et ce malgré un éventuel déplacement des cliquets 7 en position de libération hors des crémaillères 9.

Ces moyens de verrouillage bidirectionnel 33 sont plus particulièrement visibles sur la figure 12 et comprennent deux vis 34 radiales destinées à être vissées dans des logements 35 radiaux traversants des manchons d'extrémité 12 et 13. En fin de vissage, les vis 34 viennent presser les tiges 8 et 10 l'une contre l'autre, ce qui les immobilise par frottements. De façon complémentaire et facultative, un fort vissage des vis 34 peut en outre provoquer une légère déformation par expansion radiale des parties externes des manchons d'extrémité 12 et/ou 13 non recouvertes par le tube 14 : le tube 14 ne peut ainsi plus venir à recouvrement de ces parties externes de manchons par vissage. Les manchons d'extrémité 12 et 13 ne peuvent ainsi plus être rapprochés l'un de l'autre par vissage du tube 14, ce qui, du fait de l'orientation des crémaillères 9, empêche tout écartement relatif des bras 2 et 3.

Pour limiter l'encombrement du dispositif 1 avant utilisation, les bras 2 et 3 comportent des avant-bras 200 et 300 repliables par des moyens de charnière 36. Les moyens de charnière 36 autorisent un pivotement des avant-bras 200 et 300 respectivement autour de quatrième et cinquième directions IV-IV et V-V perpendiculaires aux première et deuxième directions I-I et II-II.

L'utilisation du dispositif 1 selon l'invention va désormais être décrite à l'aide des figures 1 à 12.

Avant utilisation, le dispositif 1 se trouve dans la configuration initiale illustrée sur la figure 1, dans laquelle :
- les tiges 8 et 10 sont coulissées au maximum de leur recouvrement,
- les manchons d'extrémité 12 et 13 sont au maximum de leur rapprochement,
- les avant-bras 200 et 300 sont repliés.

Dans cette configuration initiale, les moyens d'embrayage 6 maintiennent les moyens de verrouillage unidirectionnel 15 et 16 en état de débrayage grâce aux moyens de maintien 18 positionnés comme sur les figures 3 et 4.

L'opérateur commence par déplier les avant-bras 200 et 300 de façon à ce que les bras 2 et 3 se développent sensiblement selon la troisième direction III-III sur toute leur longueur.

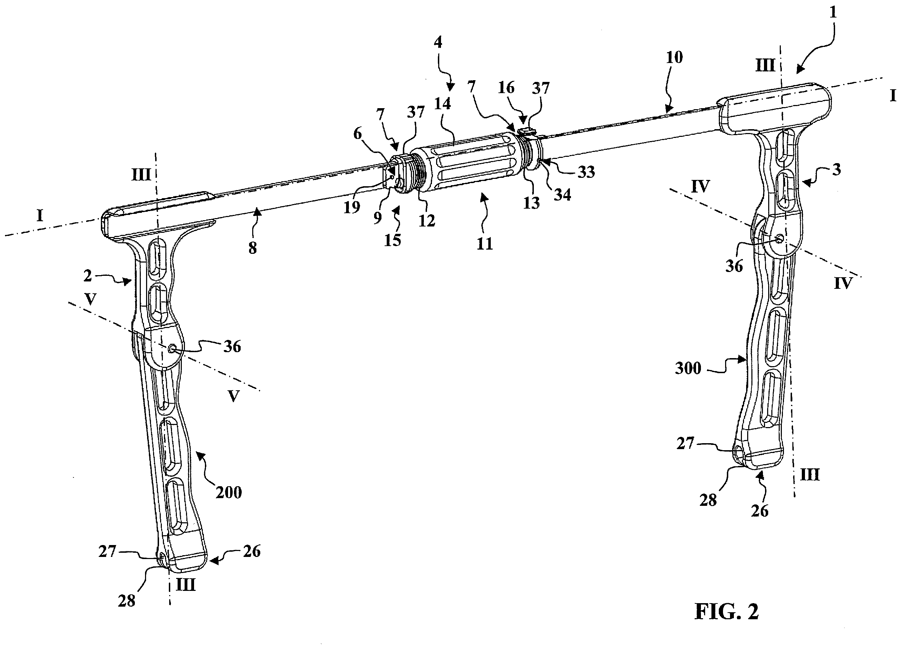

Puis, en tenant les bras 2 et 3 dans chacune de ses mains, l'opérateur leur applique une force tendant à les déplacer l'un à l'écart de l'autre pour amener le dispositif 1 dans la configuration illustrée sur la figure 2. Les moyens de verrouillage unidirectionnel 15 et 16, étant en état de débrayage, ne s'opposent pas au mouvement d'écartement provoqué par l'opérateur.

Une fois les bras 2 et 3 parvenus en position d'écartement maximal (figure 5), les ergots 19 portés par les tiges 8 et 10 déplacent les moyens de maintien 18 en position d'effacement (figure 6). Les cliquets 7 viennent alors en prise dans les crémaillères 9 des tiges 8 et 10 sous l'effet des moyens de rappel élastiques : les moyens de verrouillage unidirectionnel 15 et 16 sont alors en état d'embrayage. L'orientation des cliquets 7 et des dents des crémaillères 9 permet aux moyens de verrouillage unidirectionnel 15 et 16 d'autoriser un rapprochement relatif des bras 2 et 3 tout en s'opposant à un écartement relatif des bras 2 et 3.

Il est précisé que le déplacement des moyens de maintien 18 en position d'effacement peut se réaliser à une distance d'écartement des bras 2 et 3 prédéterminée inférieure à leur distance d'écartement maximale.

Situé d'un unique côté du patient, l'opérateur applique ensuite aux bras 2 et 3 une force tendant à les rapprocher l'un de l'autre et positionne simultanément le dispositif 1 de façon approximative au-dessus des manchons tubulaires 20 et 21. Les manchons tubulaires 20 et 21 ont préalablement été disposés à coulissement sur les broches médicales de Kirschner fixées à l'os du bassin du patient. Le dispositif 1 se trouve alors dans la configuration illustrée sur la figure 8.

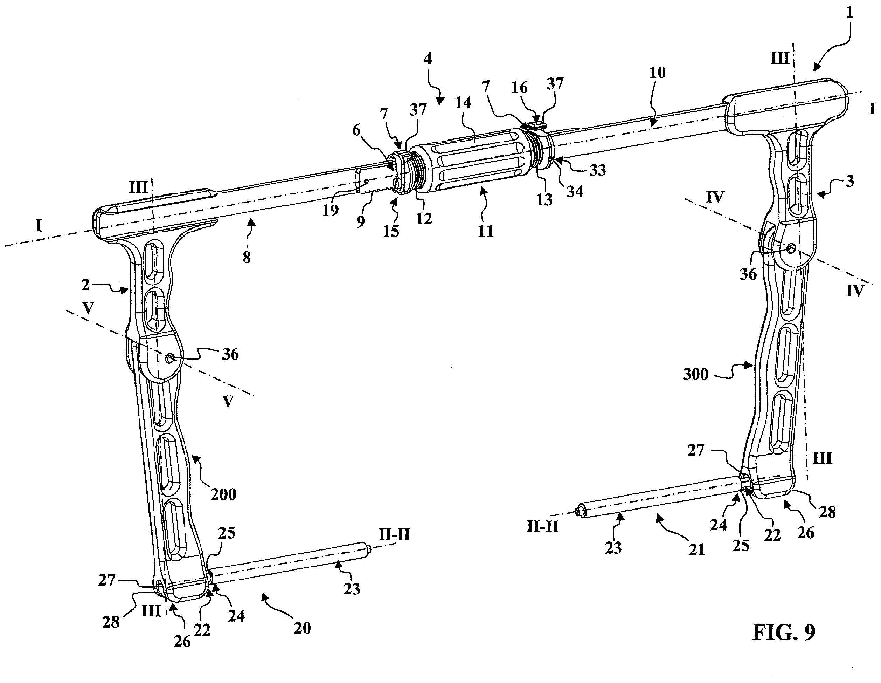

L'opérateur engage alors les premiers tronçons 22 respectifs des manchons tubulaires 20 et 21, via les fentes 28, dans les deuxièmes tronçons 30 des moyens de réception 26 (figures 9 et 10) respectifs par un simple mouvement de translation selon la troisième direction III-III. Ce mouvement de translation est facile à effectuer pour l'opérateur qui pratique une visée en ayant une vue de dessus du patient qui est allongé. Il n'y a pas besoin d'un déplacement de l'opérateur de chaque côté du patient, ni besoin de la présence de deux opérateurs.

Puis, l'opérateur poursuit le mouvement de rapprochement des bras 2 et 3 selon la première direction I-I, ce qui a pour effet de provoquer l'engagement des premiers tronçons 22 respectifs dans les premiers tronçons 29 respectifs ainsi que l'engagement des deuxièmes tronçons 23 respectifs dans les deuxièmes tronçons 30 respectifs jusqu'au contact entre les première et deuxième face de butée 25 et 32.

Les deuxièmes tronçons 30 des moyens de réception 26 viennent au contact des deuxièmes tronçons 23 des manchons tubulaires 20 et 21 respectifs selon une surface 38 supérieure à un demi-cylindre. Les manchons tubulaires 20 et 21 ne peuvent plus être sortis des moyens de réception 26 par un simple mouvement de translation selon la deuxième direction II-II.

Pour finir de rapprocher au mieux les bras 2 et 3, l'opérateur entraîne en rotation le tube 14 de façon à provoquer un éloignement relatif des premier et deuxième manchons d'extrémité 12 et 13. Du fait de la structure du dispositif 1 et de l'orientation des moyens de verrouillage unidirectionnel 15 et 16 (qui sont en état d'embrayage), l'éloignement relatif des premier et deuxième manchons d'extrémité 12 et 13 provoque un rapprochement relatif des bras 2 et 3.

Le vissage du tube 14 peut être effectué par l'opérateur unique se situant d'un côté ou de l'autre du patient, le tube 14 étant facilement accessible puisque disposé autour des moyens de glissière 4. Aucun déplacement de l'opérateur autour du patient n'est nécessaire pour achever et parfaire la mise en place du dispositif 1 sur le patient.

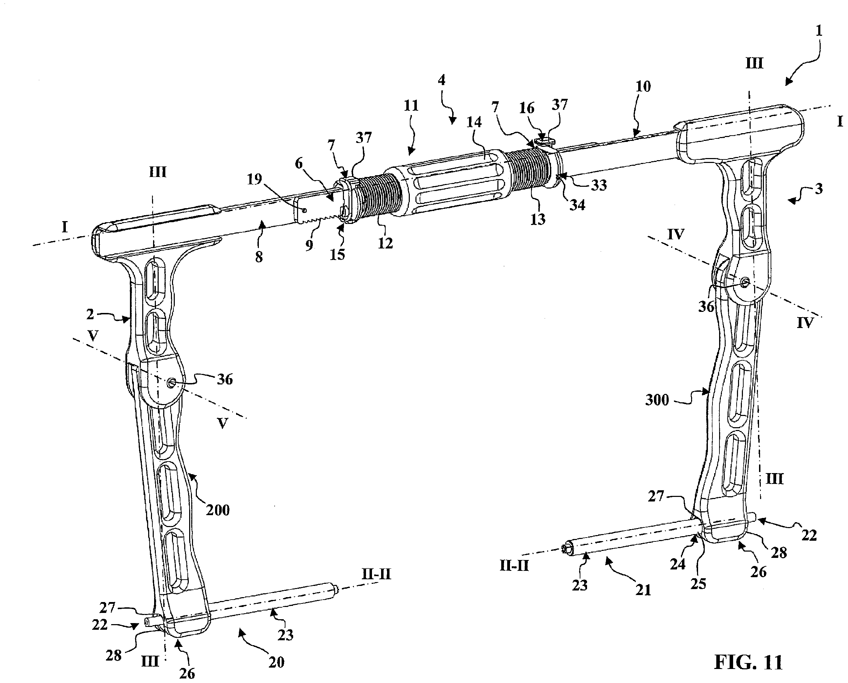

On se trouve alors dans la configuration illustrée sur la figure 11 : le dispositif 1 enserre de façon satisfaisante le bassin fracturé du patient. Les vis 34 des moyens de verrouillage 33 peuvent alors être vissées jusqu'à presser les tiges 8 et 10 l'une contre l'autre, et éventuellement déformer radialement les manchons d'extrémité 12 et 13, pour empêcher tout écartement et rapprochement relatifs des bras 2 et 3.

Lorsque le dispositif 1 n'est plus nécessaire, l'opérateur doit le retirer. L'opérateur commence par dévisser les vis 34 des moyens de verrouillage 33, puis il presse les bouton-poussoirs 37 pour déplacer les cliquets 7 des moyens de verrouillage unidirectionnel 15 et 16 en position de dégagement à rencontre des moyens de rappel élastiques : les bras 2 et 3 peuvent alors être écartés relativement l'un à l'écart de l'autre par simple traction.

Il est expressément précisé que la structure particulière des moyens d'engagement 27 et de réception 26 des bras 2 et 3 constitue la solution d'un problème technique spécifique de visée, solution pour laquelle une protection par brevet pourra être recherchée indépendamment de la structure des moyens de verrouillage unidirectionnel 15 et 16 à moyens d'embrayage 6.

De la même façon, les moyens d'entraînement 17 fins et précis des bras 2 et 3 par vissage et leur disposition constituent la solution d'un problème technique spécifique d'accessibilité pour l'opérateur, solution pour laquelle une protection par brevet pourra être recherchée indépendamment de la structure des moyens de verrouillage unidirectionnel 15 et 16 à moyens d'embrayage 6.

La présente invention n'est pas limitée aux modes de réalisation qui ont été explicitement décrits, mais elle en inclut les diverses variantes et généralisations contenues dans le domaine des revendications ci-après.

## Revendications

1. Dispositif (1) fixateur de bassin destiné à enserrer le bassin fracturé d'un patient pour le stabiliser, comprenant un premier (2) et un deuxième (3) bras disposés à coulissement par des moyens de glissière (4) permettant un éloignement relatif et un rapprochement relatif desdits bras (2, 3) selon une première direction (I-I), comportant :
- des moyens de verrouillage unidirectionnel (15, 16) du déplacement relatif des bras (2, 3), activables vers un état dit d'embrayage dans lequel les moyens de verrouillage unidirectionnel (15, 16) autorisent un rapprochement relatif des bras (2, 3) tout en s'opposant à un écartement relatif des bras (2, 3), lesdits moyens de verrouillage unidirectionnel (15, 16) comportant un cliquet (7) coopérant avec une tige (8, 10) à crémaillère (9), ledit cliquet (7) étant déplaçable entre une position d'engagement dans laquelle il est en prise dans la crémaillère (9) de la tige (8, 10) et une position de libération dans laquelle il est dégagé de la crémaillère (9),
- des moyens d'embrayage (6) des moyens de verrouillage unidirectionnel (15, 16), permettant de sélectivement activer vers l'état d'embrayage les moyens de verrouillage unidirectionnel (15, 16) depuis un état dit de débrayage dans lequel les moyens de verrouillage unidirectionnel (15, 16) autorisent un rapprochement relatif et un écartement relatif des bras (2, 3),
- des moyens de rappel élastiques rappelant en permanence le cliquet (7) vers sa position d'engagement,
**caractérisé en ce que** les moyens d'embrayage (6) comportent des moyens de maintien (18) déplaçables entre une position de maintien, dans laquelle ils maintiennent les moyens de verrouillage unidirectionnel (15, 16) en état de débrayage à l'encontre des moyens de rappel élastiques, et une position d'effacement, dans laquelle les moyens de verrouillage unidirectionnel (15, 16) peuvent librement se déplacer vers leur état d'embrayage sous l'effet des moyens de rappel élastiques.

2. Dispositif (1) selon la revendication 1, **caractérisé en ce que** les moyens d'embrayage (6) sont conformés de façon à activer les moyens de verrouillage unidirectionnel (15, 16) vers l'état d'embrayage lorsque que les bras (2, 3) ont été écartés relativement selon une distance d'écartement prédéterminée, la distance d'écartement prédéterminée étant de préférence la distance d'écartement maximale des bras (2, 3).

3. Dispositif (1) selon l'une des revendications 1 ou 2, **caractérisé en ce que** les moyens d'embrayage (6) comportent un ergot (19) porté par la tige (8, 10) à crémaillère (9) et qui, lorsque les bras (2, 3) sont écartés relativement selon une distance d'écartement prédéterminée, déplace les moyens de maintien (18) en position d'effacement.

4. Dispositif (1) selon l'une quelconque des revendications 1 à 3, **caractérisé en ce qu'**il comporte des moyens d'entraînement par vissage (17) capables de provoquer un rapprochement relatif des bras (2, 3).

5. Dispositif (1) selon l'une quelconque des revendications 1 à 4, **caractérisé en ce qu'**il comporte :
- une première tige (8) à crémaillère (9) solidaire du premier bras (2),
- une deuxième tige (10) à crémaillère (9) solidaire du deuxième bras (3),
lesdites première (8) et deuxième (10) tiges étant chacune disposée à coulissement par rapport à un manchon central (11).

6. Dispositif (1) selon la revendication 5, **caractérisé en ce que** :
- le manchon central (11) comporte un premier manchon d'extrémité (12) et un deuxième manchon d'extrémité (13) filetés extérieurement, reliés l'un à l'autre par un tube (14) creux fileté intérieurement,
- l'un des manchons d'extrémité (12, 13) comporte des premiers moyens de verrouillage unidirectionnel (15, 16) de l'une des tiges (8, 10) à crémaillère (9), tandis que l'autre des manchons d'extrémité (12, 13) comporte des deuxièmes moyens de verrouillage unidirectionnel (15, 16) de l'autre des tiges (8, 10) à crémaillère (9),
- les filetages sont conformés de façon qu'une rotation du tube (14) creux dans un premier sens provoque un rapprochement relatif des premier (12) et deuxième (13) manchons d'extrémité tandis qu'une rotation du tube (14) creux dans un deuxième sens, opposé au premier sens, provoque un éloignement relatif des premier (12) et deuxième (13) manchons d'extrémité.

7. Dispositif (1) selon l'une quelconque des revendications 1 à 6, **caractérisé en ce que** :
- il comporte des manchons tubulaires (20, 21) destinés à respectivement recevoir à coulissement une broche médicale,
- les bras (2, 3) comportent des extrémités libres à moyens de réception (26) conformés pour recevoir et tenir les manchons tubulaires (20, 21) orientés selon une deuxième direction (II-II) parallèle à la première direction (I-I),
- les extrémités libres des bras (2, 3) sont munies de moyens d'engagement (27) permettant une pénétration des manchons tubulaires (20, 21) dans les moyens de réception (26) par un mouvement de translation selon une troisième direction (III-III) perpendiculaire à la première direction (I-I).

8. Dispositif (1) selon la revendication 7, **caractérisé en ce que** les moyens d'engagement (27) comportent une fente (28) à l'extrémité libre des bras (2, 3).

9. Dispositif (1) selon l'une des revendications 7 ou 8, **caractérisé en ce que** :
- les manchons tubulaires (20, 21) comportent un premier tronçon (22) de diamètre extérieur réduit et un deuxième tronçon (23) de diamètre extérieur plus important reliés par un épaulement (24) à première face de butée (25),
- les moyens de réception (26) comportent un premier tronçon (29) de diamètre intérieur réduit et un deuxième tronçon (30) de diamètre intérieur plus important reliés par un épaulement (31) à deuxième face de butée (32),
- le premier tronçon (29) des moyens de réception (26) a un diamètre intérieur supérieur au diamètre du premier tronçon (22) de manchon tubulaire (20, 21) et inférieur au diamètre du deuxième tronçon (23) de manchon tubulaire (20, 21),
- le deuxième tronçon (30) des moyens de réception (26) a un diamètre intérieur supérieur au diamètre du deuxième tronçon (23) de manchon tubulaire (20, 21).

10. Dispositif (1) selon l'une quelconque des revendications 1 à 9, **caractérisé en ce qu'**il comporte des moyens de verrouillage (33) aptes à s'opposer à un écartement et à un rapprochement relatifs des bras (2, 3) selon la première direction (I-I).

## Patentansprüche

1. Fixiergerät (1) für ein Becken zum Einspannen des gebrochenen Beckens eines Patienten zur Stabilisierung, umfassend einen ersten (2) und einen zweiten (3) Arm, die über Linearführmittel (4) verschieblich angeordnet sind, um ein relatives Auseinanderziehen und ein relatives Aneinanderschieben der genannten Arme (2, 3) entlang einer ersten Richtung (I-I) zu ermöglichen, umfassend:
- einseitig gerichtet wirkende Sperrmittel (15, 16) zur relativen Verschiebung der Arme (2, 3), die in einem Kupplung genannte Zustand aktivierbar sind, in dem die einseitig gerichtet wirkenden Sperrmittel (15, 16) ein relatives Auseinanderschieben der Arme (2, 3) entgegen einander bis zu einem relativen Abstand der Arme (2, 3) zulassen, wobei die genannten einseitig gerichtet wirkenden Sperrmittel (15, 16) eine Sperrklinke (7) umfassen, die mit einer Zahnschienen (9)-Stange (8, 10) zusammenwirkt, wobei die genannte Sperrklinke (7) zwischen einer Eingriffsposition, in der sie auf der Zahnschienen (9)-Stange (8, 10) festgesetzt ist, und einer gelösten Position, in der sie von der Zahnschiene (9) frei ist, platzierbar ist,
- Kupplungsmittel (6) der einseitig gerichtet wirkenden Sperrmittel (15, 16), die ein selektives Aktivieren in Richtung des Kupplungszustandes der einseitig gerichtet wirkenden Sperrmittel (15, 16) bis zu einem Ausschaltung genannten Zustand gestattet, in dem die einseitig gerichtet wirkenden Sperrmittel (15, 16) ein relatives Aneinanderschieben und einen relativen Abstand der Arme (2, 3) zulassen,
- elastische Rückstellmittel, die Sperrklinke (7) ständig gegen ihre Eingriffsposition zurückstellen, **dadurch gekennzeichnet, dass** die Kupplungsmittel (6) Haltemittel (18) umfassen, die zwischen einer Halteposition, in der sie die einseitig gerichtet wirkenden Sperrmittel (15, 16) im Ausschaltungszustand gegen die elastischen Rückstellmittel halten und einer gelösten Position, in der die einseitig gerichtet wirkenden Sperrmittel (15, 16) sich frei entgegen dem Kupplungszustand unter der Wirkung der elastischen Rückstellmittel platzieren können platzierbar sind.

2. Gerät (1) nach Anspruch 1, **dadurch gekennzeichnet, dass** die Kupplungsmittel (6) in einer Weise angepasst sind, um die einseitig gerichtet wirkenden Sperrmittel (15, 16) in den Eingriffszustand zu aktivieren, wenn die Arme (2, 3) relativ mit einer vorbestimmte Abstandsdistanz verschoben worden sind, wobei die vorbestimmte Abstandsdistanz vorzugsweise die maximale Abstandsdistanz der Arme (2, 3) ist.

3. Gerät (1) nach einem der Ansprüche 1 oder 2, **dadurch gekennzeichnet, dass** die Kupplungsmittel (6) eine auf der Zahnschienen (9)-Stange (8, 10) getragenen Nocken (19) umfassen, der, wenn die Arme (2, 3) relativ mit einer vorbestimmte Abstandsdistanz verschoben sind, die Haltemittel (18) in die gelöste Position verschiebt.

4. Gerät (1) nach einem der Ansprüche 1 bis 3, **dadurch gekennzeichnet, dass** es Verschraubungsantriebsmittel (17) umfasst, die eingerichtet sind, ein relatives Aneinanderschieben der Arme (2, 3) herbeizuführen.

5. Gerät (1) nach einem der Ansprüche 1 bis 4, **dadurch gekennzeichnet, dass** es umfasst:
- eine erste Zahnschienen (9)-Stange (8), die ortsfest zum ersten Arm (2) ist,
- eine zweite Zahnschienen (9)-Stange (10), die ortsfest zum zweiten Arm (3) ist,
wobei sowohl die genannte erste (8) als auch die zweite (10) Stange auf einer zentralen Muffe (11) relativ zueinander gleitend angeordnet sind.

6. Gerät (1) nach Anspruch 5, **dadurch gekennzeichnet, dass**:
- die zentrale Muffe (11) ein erstes Muffenende (12) und ein zweites Muffenende (13) mit einem Außengewinde umfasst, die aneinander über ein Hohlrohr (14) mit einem Innengewinde gehalten sind,
- eines der Muffenenden (12, 13) erste einseitig gerichtet wirkende Sperrmittel (15, 16) an einer der Zahnschienen (9)-Stangen (8, 10) umfasst, während das andere der Muffenenden (12, 13) zweite einseitig gerichtet wirkende Sperrmittel (15, 16) an der anderen der Zahnschienen (9)-Stangen (8, 10) umfasst,
- die Gewinde derart angepasst sind, dass eine Drehung des Hohlrohres (14) in eine erste Richtung zu einem relativen Aneinanderschieben des ersten (12) und zweiten (13) Muffenendes führt, während eine Drehung des Hohlrohres (14) in eine zweite Richtung, entgegen der ersten Richtung, zu einem relativen Auseinanderziehen des ersten (12) und zweiten (13) Muffenendes führt.

7. Gerät (1) nach einem der Ansprüche 1 bis 6, **dadurch gekennzeichnet, dass**:
- es Röhrenmuffen (20, 21) zum entsprechenden Aufnehmen einer Verschiebung eines medizinischen Steckers umfasst,
- die Arme (2, 3) an freie Enden Aufnahmemittel (26) umfassen, die zum Aufnehmen und Halten der Röhrenmuffen (20, 21) angepasst und entlang einer zweiten Richtung (II-II) parallel zur ersten Richtung (I-I) ausgerichtet sind,
- wobei die freien Enden der Arme (2, 3) mit Eingriffsmitteln (27) ausgestattet sind, die einen Eingriff der Röhrenmuffen (20, 21) in die Aufnahmemittel (26) durch eine translatorische Bewegung entlang einer dritten Richtung (III-III) rechtwinklig zur ersten Richtung (I-I) erlauben.

8. Gerät (1) nach Anspruch 7, **dadurch gekennzeichnet, dass** die Eingriffmittel (27) einen Schlitz (28) an einem freien Ende der Arme (2, 3) umfassen.

9. Gerät (1) nach Anspruch 7 oder 8, **dadurch gekennzeichnet, dass**
- die Röhrenmuffen (20, 21) einen ersten Abschnitt (22) mit einem reduzierten Außendurchmesser und einen zweiten Abschnitt (23) mit einem größeren Außendurchmesser umfassen, der an einer ersten stirnseitigen (25) Schulter (24) anliegt,
- die Aufnahmemittel (26) einen ersten Abschnitt (29) mit einem reduzierten Innendurchmesser und einen zweiten Abschnitt (30) mit einem größeren Innendurchmesser umfassen, der an einer zweiten stirnseitigen (32) Schulter (31) anliegt,
- der erste Abschnitt (29) der Aufnahmemittel (26) einen Innendurchmesser besitzt, der größer ist, als ein Durchmesser des ersten Abschnittes (22) der Röhrenmuffe (20, 21) und kleiner ist, als ein Durchmesser des zweiten Abschnittes (23) der Röhrenmuffe (20, 21),
- der zweite Abschnitt (30) der Aufnahmemittel (26) einen Innendurchmesser besitzt, der größer ist, als ein Durchmesser des zweiten Abschnittes (23) der Röhrenmuffe (20, 21).

10. Gerät (1) nach einem der Ansprüche 1 bis 9, **dadurch gekennzeichnet, dass** es Sperrmittel (33) umfasst, die eingerichtet sind, in einem Abstand sich gegenüberzuliegen und in der ersten Richtung (I-I) die Arme (2, 3) aufeinander zuzuschieben.

## Claims

1. Pelvic fixator device (1) intended to clamp the fractured pelvis of a patient in order to stabilize it, comprising a first arm (2) and a second arm (3) which are arranged to be slidable by slide means (4) in order to allow said arms (2, 3) to move away from each other and move towards each other in a first direction (I-I), having:
- unidirectional locking means (15, 16) which lock the relative movement of the arms (2, 3) and which can be activated towards an engaged state in which the unidirectional locking means (15, 16) allow the arms (2, 3) to move towards each other while opposing a movement of the arms (2, 3) away from each other, said unidirectional locking means (15, 16) having a detent (7) that cooperates with a rod (8, 10) with rack (9), said detent (7) being movable between an engagement position, in which it engages in the rack (9) of the rod (8, 10), and a release position, in which it is disengaged from the rack (9),
- clutch means (6) for the unidirectional locking means (15, 16), making it possible to selectively activate the unidirectional locking means (15, 16) towards the engaged state from a disengaged state in which the unidirectional locking means (15, 16) allow said arms (2, 3) to move towards each other and move away from each other,
- elastic return means that permanently return the detent (7) to its engagement position,
**characterized in that** the clutch means (6) have holding means (18) that are movable between a holding position, in which they hold the unidirectional locking means (15, 16) in the disengaged state counter to the elastic return means, and a retracted position, in which the unidirectional locking means (15, 16) can move freely towards their engaged state under the effect of the elastic return means.

2. Device (1) according to Claim 1, **characterized in that** the clutch means (6) are designed in such a way as to activate the unidirectional locking means (15, 16) towards the engaged state when the arms (2, 3) have been moved away from each other by a predetermined spacing distance, the predetermined spacing distance preferably being the maximum spacing distance of the arms (2, 3).

3. Device (1) according to either of Claims 1 and 2, **characterized in that** the clutch means (6) have a lug (19) which is carried by the rod (8, 10) with rack (9) and, when the arms (2, 3) are moved away from each other by a predetermined spacing distance, moves the holding means (18) to the retracted position.

4. Device (1) according to any one of Claims 1 to 3, **characterized in that** it has screw-type driving means (17) capable of causing the arms (2, 3) to move towards each other.

5. Device (1) according to any one of Claims 1 to 4, **characterized in that** it has:
- a first rod (8) with rack (9), rigidly connected to the first arm (2),
- a second rod (10) with rack (9), rigidly connected to the second arm (3),
said first rod (8) and second rod (10) each being arranged slidably with respect to a central sleeve (11) .

6. Device (1) according to Claim 5, **characterized in that**:
- the central sleeve (11) has a first end sleeve (12) and a second end sleeve (13) which are threaded externally and are connected to each other by an internally threaded hollow tube (14),
- one of the end sleeves (12, 13) has first unidirectional locking means (15, 16) for one of the rods (8, 10) with rack (9), while the other of the end sleeves (12, 13) has second unidirectional locking means (15, 16) for the other of the rods (8, 10) with rack (9),
- the threads are designed in such a way that a rotation of the hollow tube (14) in a first direction causes the first end sleeve (12) and the second end sleeve (13) to move towards each other, while a rotation of the hollow tube (14) in a second direction, counter to the first direction, causes the first end sleeve (12) and the second end sleeve (13) to move away from each other.

7. Device (1) according to any one of Claims 1 to 6, **characterized in that**:
- it has tubular sleeves (20, 21) which are each intended to respectively receive a medical pin in a sliding manner,
- the arms (2, 3) have free ends with receiving means (26) designed to receive and hold the tubular sleeves (20, 21) oriented in a second direction (II-II) parallel to the first direction (I-I),
- the free ends of the arms (2, 3) are equipped with engagement means (27) allowing the tubular sleeves (20, 21) to penetrate the receiving means (26) in a translational movement in a third direction (III-III) perpendicular to the first direction (I-I).

8. Device (1) according to Claim 7, **characterized in that** the engagement means (27) have a slit (28) at the free end of the arms (2, 3).

9. Device (1) according to either of Claims 7 and 8, **characterized in that**:
- the tubular sleeves (20, 21) have a first segment (22) of reduced external diameter and a second segment (23) of greater external diameter, which are connected by a shoulder (24) with first abutment face (25),
- the receiving means (26) have a first segment (29) of reduced internal diameter and a second segment (30) of greater internal diameter, which are connected by a shoulder (31) with second abutment face (32),
- the first segment (29) of the receiving means (26) has an internal diameter greater than the diameter of the first segment (22) of tubular sleeve (20, 21) and smaller than the diameter of the second segment (23) of tubular sleeve (20, 21),
- the second segment (30) of the receiving means (26) has an internal diameter greater than the diameter of the second segment (23) of tubular sleeve (20, 21).

10. Device (1) according to any one of Claims 1 to 9, **characterized in that** it has locking means (33) that are able to counter the arms (2, 3) moving towards each other and away from each other in the first direction (I-I).
